# EUROPÄISCHE PATENTANMELDUNG

(11) **EP 3 984 483 A1**
(43) Veröffentlichungstag der Anmeldung: **20.04.2022**
(21) Anmeldenummer: 21197628.7
(22) Anmeldetag: 20.09.2021
(51) Int. Cl.: A61B 18/14, A61B 17/00, A61B 18/00, A61B 17/29

(54) **ISOLATION EINES SCHAFTS**

(30) Priorität: 14.10.2020 DE 102020126968
(71) Anmelder: Karl Storz SE & Co. KG, 78532 Tuttlingen (DE)
(72) Erfinder: Blocher, Martin, 78532 Tuttlingen (DE); Grüner, Sven Axel, 78532 Tuttlingen (DE); Holzer, Judith, 78532 Tuttlingen (DE); Kärcher, Daniel, 78532 Tuttlingen (DE); Längle, Dominik, 78532 Tuttlingen (DE); Merz, Robin, 78532 Tuttlingen (DE); Schneider, Janosz, 78532 Tuttlingen (DE); Schneider, Sven, 78532 Tuttlingen (DE); Stefan, Jochen, 78532 Tuttlingen (DE); Unger, Tobias, 78532 Tuttlingen (DE)

(57) **Zusammenfassung**

Ein elektrisch isolierter Schaft (30) eines mikroinvasiven Instruments oder für ein mikroinvasives Instrument umfasst ein elektrisch leitfähiges Schaftbauteil (40) mit einer äußeren Oberfläche (44) und ein Isolationsrohr (50) aus einem elektrisch isolierenden Material, das das Schaftbauteil (40) umschließt und die äußere Oberfläche (44) des Schaftbauteils (40) bedeckt. Das Isolationsrohr (50) ist als ursprünglich rohrförmiges Halbzeug auf die äußere Oberfläche (44) des Schaftbauteils (40) aufgeschmolzen oder aufgeschweißt.

## Beschreibung

Die vorliegende Erfindung ist auf einen elektrisch isolierten Schaft, ein mikroinvasives Instrument mit einem elektrisch isolierten Schaft und ein Verfahren zum Herstellen eines elektrisch isolierten Schafts gerichtet.

Bei elektrochirurgischen oder Hochfrequenz-chirurgischen Verfahren werden hochfrequente Wechselströme verwendet, um Gewebe zu erwärmen und damit zu verändern, insbesondere zu koagulieren, zu verschweißen oder zu durchtrennen. Die dafür erforderliche Wechselspannung und elektrische Leistung werden zumindest teilweise über elektrochirurgische Instrumente zugeführt. Um den erwünschten Stromkreis klar zu definieren, Kurzschlüsse und andere unerwünschte Strompfade und die damit einhergehenden Risiken und Schäden für Patient, medizinisches Personal und Geräte zu vermeiden, ist eine elektrische Isolation erforderlich.

Instrumente für die Anwendung in der mikroinvasiven Chirurgie (oft auch als minimalinvasive Chirurgie bezeichnet) weisen in der Regel einen langen, dünnen Schaft auf. Dieser lange und dünne Schaft soll in der Regel einen möglichst geringen Querschnitt aufweisen und für elektrochirurgische Anwendungen gleichzeitig elektrisch isoliert sein. Häufig wird eine äußere Isolationsschicht in Form eines Schrumpfschlauchs aufgebracht, der jedoch unbefriedigende mechanische Eigenschaften aufweist. Andere elektrisch isolierende Beschichtungen sind in der Regel aufwändig und entsprechend teuer.

Eine Aufgabe der vorliegenden Erfindung besteht darin, einen verbesserten elektrisch isolierten Schaft, ein verbessertes mikroinvasives Instrument mit einem elektrisch isolierten Schaft und ein verbessertes Verfahren zum Herstellen eines elektrisch isolierten Schafts zu schaffen.

Diese Aufgabe wird gelöst durch die Gegenstände der unabhängigen Ansprüche.

Weitere Ausführungsformen sind in den abhängigen Ansprüchen definiert.

Ausführungsformen der vorliegenden Erfindung beruhen auf der Idee, eine Isolationsschicht in Form eines vorgefertigten Isolationsrohres aus einem elektrisch isolierenden Material über ein elektrisch leitfähiges, nämlich insbesondere metallisches Schaftbauteil zu stülpen und danach durch Erhitzen des Schaftbauteils die innere Oberfläche des Isolationsrohres mit der äußeren Oberfläche des Schaftbauteils zu verschweißen.

Ein elektrisch isolierter Schaft eines mikroinvasiven Instruments oder für ein mikroinvasives Instrument umfasst ein elektrisch leitfähiges Schaftbauteil mit einer äußeren Oberfläche und ein Isolationsrohr aus einem elektrisch isolierenden Material, das das Schaftbauteil umschließt und die äußere Oberfläche des Schaftbauteils bedeckt, wobei das Isolationsrohr als ursprünglich rohrförmiges Halbzeug auf die äußere Oberfläche des Schaftbauteils aufgeschmolzen oder aufgeschweißt ist.

Der elektrisch isolierte Schaft kann dauerhaft Bestandteil eines nicht zerlegbaren oder nur teilweise zerlegbaren mikroinvasiven Instruments oder ein eigenständiges Bauteil, das - insbesondere ohne Verwendung von Werkzeug - von anderen Bauteilen vollständig und zerstörungsfrei trennbar und mit diesen wieder mechanisch verbindbar ist, sein. Alternativ kann der elektrisch isolierte Schaft für eine Verwendung in einem zerlegbaren oder nicht zerlegbaren mikroinvasiven Instrument vorgesehen und ausgebildet sein.

Der elektrisch isolierte Schaft ist insbesondere lang und dünn, wobei die Länge mindestens das Fünffache oder mindestens das Zehnfache oder mindestens das Zwanzigfache des Durchmessers beträgt. Das Isolationsrohr erstreckt sich insbesondere mindestens über die Hälfte oder mindestens über drei Viertel oder mindestens über neun Zehntel der Länge des elektrisch isolierten Schafts. Das Schaftbauteil kann rohrförmig mit einem Lumen oder mehreren parallel angeordneten Lumina oder als massiver Stab ausgebildet sein.

Das Isolationsrohr kann zumindest an seinen Enden in streifenförmigen Bereichen, die das Schaftbauteil ringförmig vollständig umschließen, auf die äußere Oberfläche des Schaftbauteils aufgeschmolzen oder aufgeschweißt sein. Dies kann ein Eindringen von Fluiden oder Festkörpern in einen Zwischenraum zwischen dem Isolationsrohr und der Oberfläche des Schaftbauteils verhindern. Alternativ kann das Isolationsrohr vollflächig auf die äußere Oberfläche des Schaftbauteils aufgeschmolzen oder aufgeschweißt sein.

Das Aufschmelzen oder Aufschweißen des Isolationsrohrs, das also bereits ursprünglich als rohrförmiges Halbzeug vorliegt, auf die Oberfläche des Schaftbauteils kann eine robuste mechanische Verbindung des Isolationsrohrs als Isolationsschicht mit dem Schaftbauteil ermöglichen. Die stoffschlüssige Verbindung zwischen Isolationsrohr und Oberfläche des Schaftbauteils kann dabei auch mit deutlich geringerem Aufwand als beispielsweise eine Verklebung erfolgen. Die Bildung einer Isolationsschicht aus ursprünglich rohrförmigem Halbzeug ermöglicht ferner die Verwendung elektrisch isolierender Materialien, die beispielsweise in Vergleich zu einem Schrumpfschlauch deutlich vorteilhafte mechanische Eigenschaften aufweisen.

Ein mikroinvasives Instrument umfasst einen Schaft, wie er hier beschrieben ist, eine Handhabungseinrichtung an einem proximalen Ende des Schafts zur manuellen Führung und Steuerung des mikroinvasiven Instruments und ein Werkzeug an einem distalen Ende des Schafts.

Das mikroinvasive Instrument ist beispielsweise zum Greifen, Quetschen, Verschließen und/oder Durchtrennen eines Gefäßes oder anderen Gewebes vorgesehen und ausgebildet. Dazu weist das Werkzeug am distalen Ende des mikroinvasiven Instruments insbesondere zwei oder mehr Maulteile oder Branchen auf, die relativ zu einander bewegbar sind und beispielsweise Greifflächen oder Schneidkanten aufweisen.

Ein mikroinvasives Instrument, wie es hier beschrieben ist, ist insbesondere als elektrochirurgisches Instrument ausgebildet, wobei das Schaftbauteil Teil eine Stromkreises zur Übertragung von elektrischer Leistung oder eines elektrischen Signals ist.

Das Schaftbauteil, das vor allem mechanische Funktionen erfüllt, ist also gleichzeitig als Teil eines Stromkreises vorgesehen und ausgebildet, um eine weitere elektrische Leitung einzusparen und einen kleineren Querschnitt zu ermöglichen. Das proximale Ende oder ein proximaler Bereich des Schaftbauteils ist mit einem elektrischen Steckkontakt oder einem anderen elektrisch leitfähigen Anschluss am proximalen Ende des mikroinvasiven Instruments elektrisch leitfähig verbunden. Das distale Ende oder ein distaler Bereich des Schaftbauteils ist insbesondere mit einem oder mehreren Maulteilen oder Branchen des Werkzeugs elektrisch leitfähig verbunden.

Ein Verfahren zum Herstellen eines elektrisch isolierten Schafts für ein mikroinvasives Instrument umfasst ein Bereitstellen eines Schaftbauteils mit einer äußeren Oberfläche, ein Bereitstellen eines Isolationsrohrs aus einem elektrisch isolierenden Material, ein Anordnen des Schaftbauteils in dem Isolationsrohr, ein Erwärmen des Schaftbauteils bis zu einer Schmelztemperatur oder einer Übergangstemperatur oder einer Erweichungstemperatur des elektrisch isolierenden Materials des Isolationsrohrs und ein Abkühlen des Schaftbauteils und des Isolationsrohrs.

Das Verfahren ist insbesondere zum Herstellen eines elektrisch isolierten Schafts, wie er hier beschrieben ist, geeignet und vorgesehen. Ein elektrisch isolierter Schaft, wie er hier beschrieben ist, wird insbesondere mittels eines Verfahrens, wie es hier beschrieben ist, hergestellt.

Die oben beschriebenen Verfahrensschritte werden in der beschriebenen Reihenfolge ausgeführt. Insbesondere wird das Isolationsrohr aus dem elektrisch isolierenden Material als rohrförmiges Halbzeug bereitgestellt, bevor das Schaftbauteil in dem Isolationsrohr angeordnet wird. Das Isolationsrohr wird also nicht erst auf der äußeren Oberfläche des Schaftbauteils gebildet.

Der Querschnitt des Lumens des Isolationsrohrs und der Querschnitt des Schaftbauteils sind insbesondere so gewählt, dass zwischen der inneren Oberfläche des Isolationsrohrs und der äußeren Oberfläche des Schaftbauteils kein oder nur ein sehr schmaler Spalt verbleibt. Das Schaftbauteil wird insbesondere nur so weit und nur so lang erwärmt, dass die innere Oberfläche des Isolationsrohrs aufschmilzt und nach dem Abkühlen mit der äußeren Oberfläche des Schaftbauteils eine stoffschlüssige Schweißverbindung eingeht.

Bei einem Verfahren, wie es hier beschrieben ist, wird insbesondere ferner vor dem Anordnen des Schaftbauteils in dem Isolationsrohr die äußere Oberfläche des Schaftbauteils durch Bestrahlen mit einem festen Strahlmittel behandelt.

Das Bestrahlen mit einem festen Strahlmittel wird umgangssprachlich auch als Sandstrahlen, fachsprachlich auch als Druckluftstrahlen mit festem Strahlmittel und im Englischen als Sandblasting oder Abrasive Blasting bezeichnet. Das Strahlmittel liegt dabei feinkörnig in festem Aggregatszustand vor. Als Strahlmittel wird beispielsweise Sand, Hochofenschlacke, Glasgranulat, Korund, Stahl, Kunststoffgranulat, Nussschalen oder Soda in geeigneten Mahlgraden verwendet.

Das Bestrahlen mit einem festen Strahlmittel kann die Rauhigkeit und damit den Flächeninhalt der äußeren Oberfläche des Schaftbauteils vergrößern und die stoffschlüssige Verbindung zwischen dem Isolationsrohr und dem Schaftbauteil verbessern.

Bei einem Verfahren, wie es hier beschrieben ist, wird insbesondere ferner vor dem Anordnen des Schaftbauteils in dem Isolationsrohr die äußere Oberfläche des Schaftbauteils entfettet.

Das Entfetten der äußeren Oberfläche des Schaftbauteils erfolgt insbesondere mittels eines Plasmas. Alternativ kann beispielsweise ein Lösungsmittel verwendet werden.

Bei einem Verfahren, wie es hier beschrieben ist, wird insbesondere ferner vor dem Anordnen des Schaftbauteils in dem Isolationsrohr die innere Oberfläche des Isolationsrohrs entfettet.

Das Entfetten der inneren Oberfläche des Isolationsrohrs erfolgt insbesondere mittels Plasma. Alternativ kann beispielsweise ein Lösungsmittel verwendet werden, das das elektrisch isolierende Material des Isolationsrohrs nicht löst.

Bei einem Verfahren, wie es hier beschrieben ist, umfasst das Erwärmen des Schaftbauteils insbesondere ein Erzeugen eines orts- und zeitabhängigen Temperaturprofils entlang des Schaftbauteils mit einem Maximum bei oder über einer Schmelztemperatur oder einer Übergangstemperatur oder einer Erweichungstemperatur des elektrisch isolierenden Materials des Isolationsrohrs, wobei die Position des Maximums des Temperaturprofils entlang des Schaftbauteils bewegt wird.

Die Schmelztemperatur oder Übergangstemperatur oder Erweichungstemperatur des elektrisch isolierenden Materials des Isolationsrohrs wird also an jedem Ort nur innerhalb einer vorbestimmten Zeitdauer überschritten, wobei der Bereich, in dem die Schmelztemperatur, die Übergangstemperatur oder die Erweichungstemperatur erreicht oder überschritten ist, auf vorbestimmte Weise entlang dem Schaftbauteil fortbewegt wird. Der Bereich, in dem die Schmelztemperatur oder Übergangstemperatur oder Erweichungstemperatur erreicht oder überschritten ist, wird insbesondere von einem Ende des Schaftbauteils mit einer sich verändernden oder konstanten Geschwindigkeit zu dem anderen Ende des Schaftbauteils bewegt. Alternativ wird die Schmelztemperatur oder Übergangstemperatur oder Erweichungstemperatur beispielsweise zunächst in einem mittleren Bereich des Schaftbauteils erreicht oder überschritten, wobei von diesem mittleren Bereich ausgehend nacheinander oder gleichzeitig zwei Bereiche, in denen die Schmelztemperatur oder Übergangstemperatur oder Erweichungstemperatur erreicht oder überschritten ist, zu den Enden des Schaftbauteils bewegt werden.

Das beschriebene zeit- und ortsabhängige Erreichen oder Überschreiten der Schmelztemperatur oder Übergangstemperatur oder Erweichungstemperatur des elektrisch isolierenden Materials bewirkt, dass nicht das gesamte Isolationsrohr gleichzeitig mit der Oberfläche des Schaftbauteils verschweißt oder verschmolzen wird, sondern der Ort des Verschweißens oder Verschmelzens in einer vorbestimmten Weise die gesamte Oberfläche des Schaftbauteils überstreicht. Dies kann beispielsweise der Bildung von Gasblasen vorbeugen und eine restlos vollflächige Verbindung des Isolationsrohrs mit dem Schaftbauteil begünstigen.

Bei einem Verfahren, wie es hier beschrieben ist, umfasst das Heizen des Schaftbauteils insbesondere ein Erzeugen eines Wirbelstroms in dem Schaftbauteil.

Der Wirbelstrom wird insbesondere mittels einer Induktionsspule, die das Schaftbauteil umschließt, erzeugt. Mit einer Induktionsspule, die kürzer oder viel kürzer als das Schaftbauteil ist, kann an der äußeren Oberfläche des Schaftbauteils ein Temperaturprofil mit einem Maximum erzeugt werden. Durch Bewegen des Schaftbauteils relativ zu der Induktionsspule oder der Induktionsspule relativ zu dem Schaftbauteil kann dieses Temperaturmaximum entlang dem Schaftbauteil bewegt werden.

Bei einem Verfahren, wie es hier beschrieben ist, ist das Schaftbauteil insbesondere ein Schaftrohr mit einem Lumen, wobei das Erwärmen des Schaftbauteils ein Zuführen von Leistung zu einer in dem Lumen des Schaftrohrs angeordneten Heizeinrichtung und ein Bewegen der Heizeinrichtung relativ zu dem Schaftrohr entlang des Schaftrohrs umfasst.

Die Heizeinrichtung umfasst beispielsweise einen elektrischen Heizwiderstand in einem zylindrischen Gehäuse, ähnlich wie eine Heizpatrone für andere Anwendungen. Durch ein Bewegen der Heizeinrichtung entlang des Schaftrohrs kann ein orts- und zeitabhängiges Temperaturprofil mit einem Maximum bei oder über einer Schmelztemperatur oder einer Übergangstemperatur oder einer Erweichungstemperatur des elektrisch isolierenden Materials des Isolationsrohrs erzeugt werden.

### Kurzbeschreibung der Figuren

Nachfolgend werden Ausführungsformen anhand der beigefügten Figuren näher erläutert. Es zeigen:
- Figur 1: eine schematische Darstellung eines mikroinvasiven elektrochirurgischen Instruments;
- Figur 2: eine schematische Darstellung eines Querschnitts durch einen Schaft des mikroinvasiven elektrochirurgischen Instruments aus Figur 1;
- Figur 3: eine schematische Darstellung von Querschnitten von Bauteilen des in Figur 2 dargestellten Schafts;
- Figur 4: eine schematische Darstellung der in Figur 3 gezeigten Bauteile vor einem Zusammensetzen;
- Figur 5: eine schematische Darstellung eines Längsschnitts durch den Schaft aus den Figuren 1 bis 4 und eines Temperaturprofils;
- Figur 6: eine weitere schematische Darstellung eines Längsschnitts durch den Schaft aus den Figuren 1 bis 5 und eines Temperaturprofils;
- Figur 7: ein schematisches Flussdiagramm eines Verfahrens zum Herstellen eines elektrisch isolierten Schafts.

### Beschreibung der Ausführungsformen

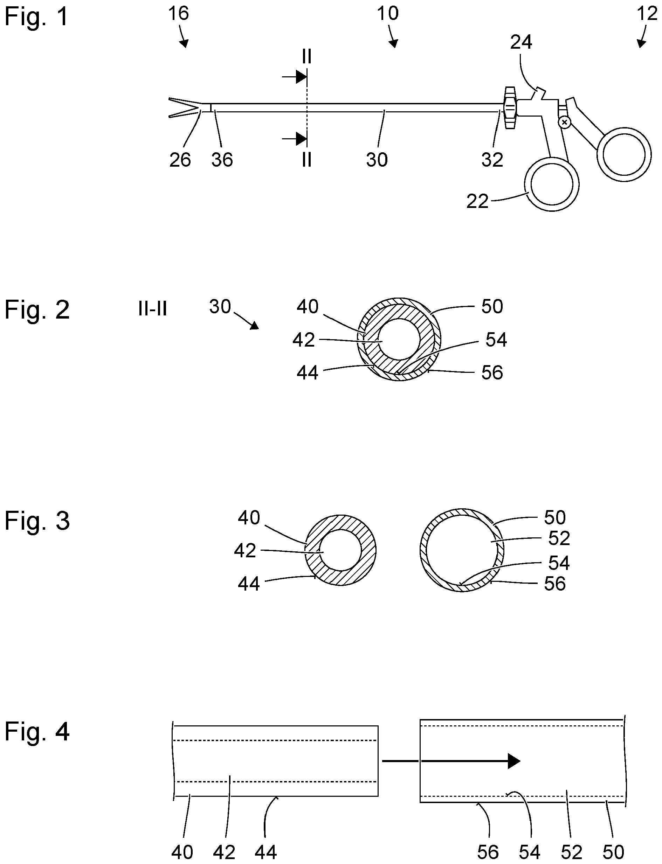

Figur 1 zeigt eine schematische Darstellung eines mikroinvasiven elektrochirurgischen Instruments 10 mit einem proximalen Ende 12 und einem distalen Ende 16. Das proximale Ende 12 des mikroinvasiven elektrochirurgischen Instruments 10 wird durch eine Handhabungseinrichtung 22 gebildet. Bei dem dargestellten Beispiel umfasst die Handhabungseinrichtung mehrere relativ zueinander bewegbare Griffteile, die eine manuelle Steuerung einer oder mehrerer Wirkfunktionen des mikroinvasiven elektrochirurgischen Instruments 10 ermöglichen. An der Handhabungseinrichtung 22 ist ein elektrischer Steckkontakt 24 zur Zuführung elektrischer Leistung vorgesehen.

Das distale Ende 16 des mikroinvasiven elektrochirurgischen Instruments 10 wird durch ein Werkzeug 26 gebildet, bei dem dargestellten Beispiel ein Greif-, Quetsch- und/oder SchneidWerkzeug. Das mikroinvasive elektrochirurgische Instrument 10 umfasst ferner einen Schaft 30, dessen proximales Ende 32 mit der Handhabungseinrichtung 22 und dessen distales Ende 36 mit dem Werkzeug 26 verbunden sind.

Figur 2 zeigt eine schematische und gegenüber Figur 1 vergrößerte Darstellung eines Querschnitts durch den Schaft 30 des in Figur 1 gezeigten mikroinvasiven elektrochirurgischen Instruments 10. Die Schnittebene II-II der Figur 2 ist orthogonal zu der Zeichenebene der Figur 1. Die Schnittebene II-II der Figur 2 ist in Figur 1 angedeutet.

Der Schaft 30 umfasst ein elektrisch leitfähiges Schaftbauteil 40, das bei dem dargestellten Beispiel als Schaftrohr mit einem Lumen 42 ausgebildet ist. In dem Lumen 42 kann beispielsweise eine - in Figur 2 nicht dargestellte - Kraftübertragungseinrichtung zum Übertragen einer Kraft von der Handhabungseinrichtung 22 zu einem oder mehreren beweglichen Maulteilen des Werkzeugs 26 des mikroinvasiven elektrochirurgischen Instruments 10 (vgl. Figur 1) angeordnet sein.

Der Schaft 30 umfasst ferner ein Isolationsrohr 50 aus einem elektrisch isolierenden Material. Im Querschnitt umschließt das Isolationsrohr 50 das Schaftbauteil 40 vollständig. Eine innere Oberfläche 54 des Isolationsrohrs 50 ist vollflächig mit einer äußeren Oberfläche 44 des Schaftbauteils 40 verschweißt oder verschmolzen. Eine äußere Oberfläche 56 des Isolationsrohrs 50 bildet die äußere Oberfläche des Schafts 30.

Figur 3 zeigt eine schematische und gegenüber Figur 1 vergrößerte Darstellung von Querschnitten von Bauteilen des Schafts 30 aus den Figuren 1 und 2, nämlich des Schaftbauteils 40 und des Isolationsrohrs 50, vor einem Zusammensetzen. Die Schnittebenen der Figur 3 entsprechen der Schnittebene II-II der Figur 2 oder sind zu dieser parallel.

Nicht nur das Schaftbauteil 40 aus dem elektrisch leitfähigen Material, beispielsweise chirurgischem Stahl, sondern auch das Isolationsrohr 50 liegt bereits vor dem Zusammensetzen zu dem in den Figuren 1 und 2 gezeigten Schaft 30 als einstückiger Festkörper, nämlich Rohr vor. Das Isolationsrohr 50 umschließt ein Lumen 52, dessen Querschnitt weitgehend oder vollständig dem äußeren Querschnitt des Schaftbauteils 40 entspricht, und in das das Schaftbauteil 40 eingeführt werden wird.

Figur 4 zeigt eine weitere schematische Darstellung des Schaftbauteils 40 und des Isolationsrohrs 50 vor dem Einführen des Schaftbauteils 40 in das Lumen 52 des Isolationsrohrs 50. Die Zeichenebene der Figur 4 ist orthogonal zu den Zeichenebenen der Figuren 2 und 3 und parallel zu der Zeichenebene der Figur 1. Die Kontur des Lumens 42 des als Schaftrohr ausgebildeten Schaftbauteils 40 und die Kontur des Lumens 52 des Isolationsrohrs 50 sind jeweils durch gestrichelte Linien angedeutet. Durch einen Pfeil ist die zum Einführen des Schaftbauteils 40 in das Lumen 52 des Isolationsrohrs 50 erforderliche Relativbewegung angedeutet.

Vor dem Einführen des Schaftbauteils 40 in das Lumen 52 des Isolationsrohrs 50 können die äußere Oberfläche 44 des Schaftbauteils 40 und die innere Oberfläche 54 des Isolationsrohrs 50 vorbehandelt werden. Insbesondere wird die äußere Oberfläche 44 des Schaftbauteils 40 mit einem feinkörnigen festen Strahlmittel bestrahlt. Dadurch kann die äußere Oberfläche 44 des Schaftbauteils 40 aufgeraut und ihr Flächeninhalt vergrößert werden. Danach kann die äußere Oberfläche 44 des Schaftbauteils 40 entfettet werden, beispielsweise durch Einwirkung eines Plasmas. Auch die innere Oberfläche 54 des Isolationsrohrs 50 kann vor dem Einführen des Schaftbauteils 40 in das Lumen 52 des Isolationsrohrs 50 entfettet werden, beispielsweise durch Einwirkung eines Plasmas.

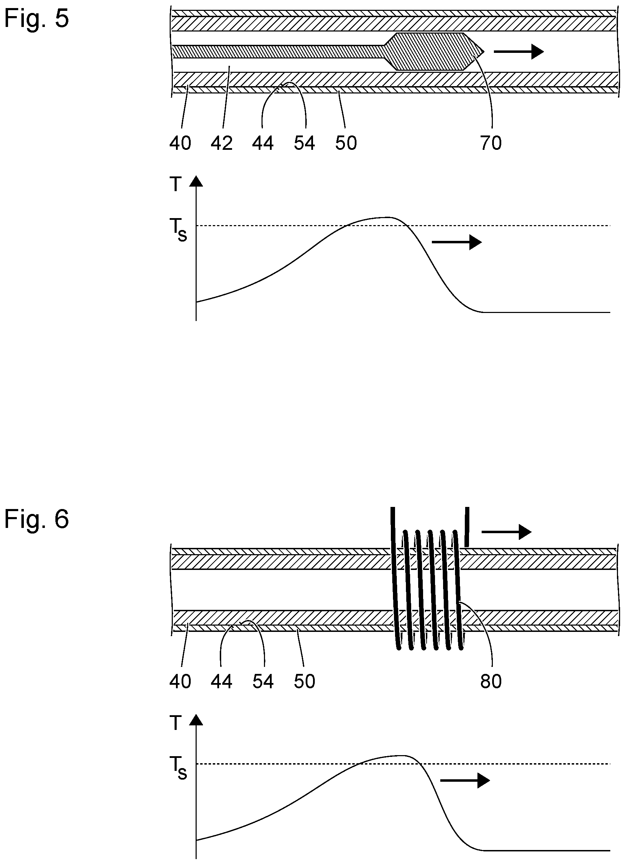

Figur 5 zeigt eine schematische Darstellung eines Schnitts durch das Schaftbauteil 40 und das Isolationsrohr 50 aus den Figuren 1 bis 4 nach dem Einführen des Schaftbauteils 40 in das Isolationsrohr 50. Die Schnittebene der Figur 5 ist parallel zu den Zeichenebenen der Figuren 1 und 4, orthogonal zu den Schnittebenen der Figuren 2 und 3 und enthält die Symmetrieachse des Schaftbauteils 40 und des Isolationsrohrs 50

Eine Heizpatrone 70 ist in dem Lumen 42 des als Schaftrohr ausgebildeten Schaftbauteils 40 angeordnet. Die Heizpatrone 70 umfasst einen elektrischen Heizwiderstand in einem zylindrischen Gehäuse, das reibungsarm in dem Lumen 42 des Schaftbauteils 40 bewegt werden kann. Während der Heizpatrone 70 elektrische Leistung zugeführt wird, wird die Heizpatrone 70 gleichzeitig wie in Figur 5 durch einen Pfeil angedeutet durch das Lumen 42 des als Schaftrohr ausgebildeten Schaftbauteils 40 bewegt. Dadurch wird an der äußeren Oberfläche 44 des Schaftbauteil 40 ein Temperaturprofil erzeugt, das in Figur 5 unten angedeutet ist. Der Ordinate ist die Temperatur T zugeordnet. Die Schmelztemperatur Ts des elektrisch isolierenden Materials des Isolationsrohrs 50 ist durch eine gestrichelte horizontale Linie angedeutet.

Die Temperatur des Schaftbauteils 40 ist - wie in Figur 6 unten rechts erkennbar, ursprünglich niedrig, insbesondere deutlich niedriger als die Schmelztemperatur Ts. Die Leistungsabgabe der Heizpatrone 70 erhöht die Temperatur T bis über die Schmelztemperatur Ts. Nach dem Passieren der Heizpatrone 70 kühlt das Schaftbauteil 40 wieder ab und unterschreitet dabei rasch die Schmelztemperatur Ts. In dem kurzen Zeitraum, in dem die Temperatur T an der äußeren Oberfläche 44 des Schaftbauteils 40 und damit auch die Temperatur des Isolationsrohrs 50 an dessen innerer Oberfläche 54 über der Schmelztemperatur Ts liegt, benetzt das elektrisch isolierende Material des Isolationsrohrs 50 die äußere Oberfläche 44 des Schaftbauteils 40. Beim Abkühlen der äußeren Oberfläche 44 des Schaftbauteils 40 und damit auch der inneren Oberfläche 54 des Isolationsrohrs 50 unter die Schmelztemperatur Ts erstarrt das elektrisch isolierende Material des Isolationsrohrs 50 wieder. Dadurch wird eine stoffschlüssige Verbindung des Isolationsrohrs 50 mit dem Schaftbauteil 40 gebildet.

Figur 6 zeigt eine schematische Darstellung eines weiteren Längsschnitts durch das Schaftbauteil 40 und das Isolationsrohr 50 aus den Figuren 2 bis 5. Die Art der Darstellung, insbesondere die Lage der Schnittebene, entspricht derjenigen der Figur 5. Auch das Schaftbauteil 40 und das Isolationsrohr 50 entsprechen den anhand der Figuren 2 bis 5 dargestellten.

In Figur 6 ist eine alternative Form der Erhitzung der äußeren Oberfläche 44 des Schaftbauteils 40 gezeigt, nämlich mittels einer Induktionsspule 80. Das Schaftbauteil 40 und das Isolationsrohr 50 sind in der Induktionsspule 80 angeordnet, die Induktionsspule 80 umgibt das Schaftbauteil 40 und das Isolationsrohr 50 ringförmig. Ein Wechselstrom in der Induktionsspule 80 induziert in dem Schaftbauteil 40 nahe dessen äußerer Oberfläche 44 einen Wirbelstrom, der aufgrund des elektrischen Widerstands des Schaftbauteils 40 dieses ohmsch erwärmt. Das Schaftbauteil 40 und das Isolationsrohr 50 einerseits und die Induktionsspule andererseits werden - wie in Figur 6 durch einen Pfeil angedeutet - relativ zu einander bewegt. Es entsteht ein ähnliches Temperaturprofil wie oben anhand der Figur 5 beschrieben.

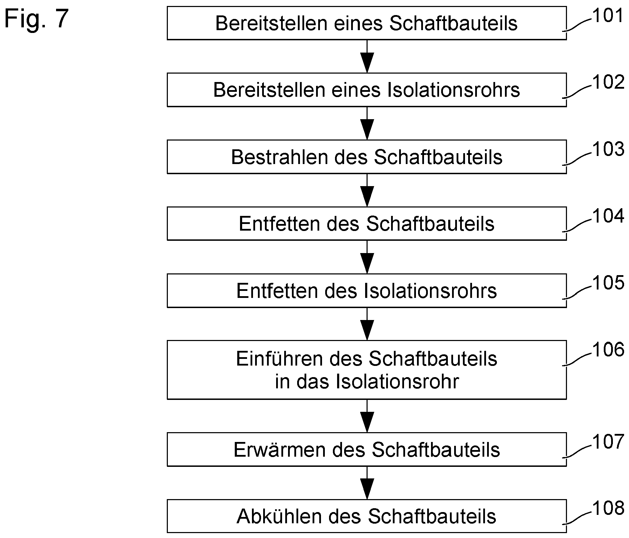

Figur 7 zeigt ein schematisches Flussdiagramm eines Verfahrens zum Herstellen eines elektrisch isolierten Schafts. Das Verfahren ist insbesondere zur Herstellung eines Schafts mit anhand der Figuren 1 bis 6 dargestellten Merkmalen und Eigenschaften anwendbar. Deshalb werden nachfolgend beispielhaft Bezugszeichen aus den Figuren 1 bis 6 verwendet.

Bei einem ersten Schritt 101 wird ein elektrisch leitfähiges Schaftbauteil 40, insbesondere ein Schaftrohr bereitgestellt. Das Schaftbauteil 40 ist beispielsweise aus chirurgischem Stahl gefertigt. Bei einem zweiten Schritt 102 wird ein Isolationsrohr 50 aus einem elektrisch isolierenden Material bereitgestellt. Die äußere Oberfläche des Schaftbauteils 40 und die innere Oberfläche des Isolationsrohrs 50 weisen insbesondere die gleiche Gestalt auf oder sind sehr ähnlich. Beispielsweise sind die äußere Oberfläche 44 des Schaftbauteils 40 und die innere Oberfläche 54 des Isolationsrohrs 50 jeweils kreiszylindrisch mit gleichen oder nur geringfügig unterschiedlichen Durchmessern.

Bei einem optionalen dritten Schritt 103 wird die äußere Oberfläche 44 des Schaftbauteils 40 mit einem feinkörnigen festen Strahlmittel bestrahlt. Dabei wird die äußere Oberfläche 44 des Schaftbauteils 40 insbesondere aufgeraut.

Bei einem optionalen vierten Schritt 104 wird die äußere Oberfläche 44 des Schaftbauteils 40 entfettet. Dies erfolgt insbesondere mittels eines Plasmas. Alternativ kann ein Lösungsmittel oder ein Tensid verwendet werden.

Bei einem optionalen fünften Schritt 105 wird die innere Oberfläche 54 des Isolationsrohrs 50 entfettet. Dies erfolgt insbesondere mittels eines Plasmas. Alternativ kann ein Lösungsmittel, das das elektrisch isolierende Material des Isolationsrohrs 50 nicht löst, oder ein Tensid verwendet werden.

Bei einem sechsten Schritt 106 wird das Schaftbauteil 40 in das Lumen 52 des Isolationsrohrs 50 eingeführt.

Bei einem siebten Schritt 107 wird die äußere Oberfläche 44 des Schaftbauteils 40 bis zu einer Maximaltemperatur bei oder über einer Schmelztemperatur oder einer Übergangstemperatur oder einer Erweichungstemperatur des elektrisch isolierenden Materials des Isolationsrohrs 50 erwärmt. Damit wird auch die innere Oberfläche 54 des Isolationsrohrs 50 auf dieselbe Temperatur erwärmt. Das elektrisch isolierende Material des Isolationsrohrs 50 schmilzt oder wird weich oder fließfähig und benetzt die äußere Oberfläche 44 des Schaftbauteils 40.

Das Erwärmen 107 der äußeren Oberfläche 44 des Schaftbauteils 40 erfolgt beispielsweise durch einen Wirbelstrom, der durch ein magnetisches Wechselfeld induziert wird. Wenn das Schaftbauteil 40 ein Schaftrohr mit einem Lumen 42 ist, kann alternativ beispielsweise eine Heizpatrone in dem Lumen 42 angeordnet werden. Um die Entstehung von Gasblasen zu verhindern, erfolgt das Erwärmen der äußeren Oberfläche 44 des Schaftbauteils 40 insbesondere nicht gleichzeitig, sondern beispielsweise von einem Ende des Schaftbauteils 40 zu dessen anderem Ende fortschreitend.

Bei einem achten Schritt 108 wird die äußere Oberfläche 44 des Schaftbauteils 40 und damit auch die innere Oberfläche 54 des Isolationsrohrs 50 abgekühlt. Dabei unterschreitet die Temperatur des elektrisch isolierenden Materials an der inneren Oberfläche 54 des Isolationsrohrs 50 die Schmelztemperatur oder Übergangstemperatur oder Erweichungstemperatur, und das elektrisch isolierende Material des Isolationsrohrs 50 erstarrt wieder vollständig, jedoch in vollflächiger stoffschlüssiger Verbindung mit der äußeren Oberfläche 44 des Schaftbauteils 40.

### Bezugszeichen

- 10: mikroinvasives elektrochirurgisches Instrument
- 12: proximales Ende des mikroinvasiven elektrochirurgischen Instruments 10
- 16: distales Ende des mikroinvasiven elektrochirurgischen Instruments 10

- 22: Handhabungseinrichtung am proximalen Ende 12 des mikroinvasiven elektrochirurgischen Instruments 10
- 24: elektrischer Steckkontakt an der Handhabungseinrichtung 22
- 26: Werkzeug am distalen Ende 16 des mikroinvasiven elektrochirurgischen Instruments 10

- 30: Schaft des mikroinvasiven elektrochirurgisches Instruments 10
- 32: proximales Ende des Schafts 30
- 36: distales Ende des Schafts 30

- 40: elektrisch leitfähiges Schaftrohr als Schaftbauteil des Schafts 30
- 42: Lumen des Schaftrohrs 40
- 44: äußere Oberfläche des Schaftrohrs 40

- 50: Isolationsrohr als Isolationsschicht des Schafts 30
- 52: Lumen des Isolationsrohrs 50
- 54: innere Oberfläche des Isolationsrohrs 50
- 56: äußere Oberfläche des Isolationsrohrs 50

- 70: Heizpatrone

- 80: Induktionsspule

- 101: erster Schritt (Bereitstellen Schaftbauteil)
- 102: zweiter Schritt (Bereitstellen eines Isolationsrohr)
- 103: dritter Schritt (Bestrahlen der äußeren Oberfläche des Schaftbauteils)
- 104: vierter Schritt (Entfetten der äußeren Oberfläche des Schaftbauteils)
- 105: fünfter Schritt (Entfetten der inneren Oberfläche des Isolationsrohrs)
- 106: sechster Schritt (Einführen des Schaftbauteils in das Isolationsrohr)
- 107: siebter Schritt (Erwärmen des Schaftbauteils)
- 108: achter Schritt (Abkühlen des Schaftbauteils)

## Patentansprüche

1. Elektrisch isolierter Schaft (30) eines mikroinvasiven Instruments (10) oder für ein mikroinvasives Instrument (10), mit:
einem elektrisch leitfähigen Schaftbauteil (40) mit einer äußeren Oberfläche (44);
einem Isolationsrohr (50) aus einem elektrisch isolierenden Material, das das Schaftbauteil (40) umschließt und die äußere Oberfläche (44) des Schaftbauteils (40) bedeckt,
wobei das Isolationsrohr (50) als ursprünglich rohrförmiges Halbzeug auf die äußere Oberfläche (44) des Schaftbauteils (40) aufgeschmolzen oder aufgeschweißt ist.

2. Mikroinvasives Instrument (10) mit:
einem Schaft (30) nach einem der vorangehenden Ansprüche;
einer Handhabungseinrichtung (20) an einem proximalen Ende (32) des Schafts (30) zur manuellen Führung und Steuerung des mikroinvasiven Instruments (10);
einem Werkzeug (26) an einem distalen Ende (36) des Schafts (30).

3. Mikroinvasives Instrument (10) nach dem vorangehenden Anspruch, wobei
das mikroinvasive Instrument als elektrochirurgisches Instrument ausgebildet ist,
das Schaftbauteil (30) Teil eines Stromkreises zur Übertragung von elektrischer Leistung oder eines elektrischen Signals ist.

4. Verfahren zum Herstellen eines elektrisch isolierten Schafts (30) für ein mikroinvasives Instrument (10), mit folgenden Schritten:
Bereitstellen (101) eines Schaftbauteils (40) mit einer äußeren Oberfläche (44);
Bereitstellen (102) eines Isolationsrohrs (50) aus einem elektrisch isolierenden Material;
Anordnen (106) des Schaftbauteils (40) in dem Isolationsrohr (50);
Erwärmen des Schaftbauteils (40) bis zu einer Schmelztemperatur oder einer Übergangstemperatur oder einer Erweichungstemperatur des elektrisch isolierenden Materials des Isolationsrohrs (50);
Abkühlen des Schaftbauteils (40) und des Isolationsrohrs (50).

5. Verfahren nach Anspruch 4, ferner mit folgendem Schritt vor dem Anordnen (106) des Schaftbauteils (40) in dem Isolationsrohr (50):
Behandeln (103) der äußeren Oberfläche (44) des Schaftbauteils (40) durch **Bestrahlen** mit einem festen Strahlmittel.

6. Verfahren nach einem der Ansprüche 4 und 5, ferner mit folgendem Schritt vor dem Anordnen (106) des Schaftbauteils (40) in dem Isolationsrohr (50):
**Entfetten** (104) der äußeren Oberfläche (44) des Schaftbauteils (40).

7. Verfahren nach einem der Ansprüche 4 bis 6, ferner mit folgendem Schritt vor dem Anordnen (106) des Schaftbauteils 40 in dem Isolationsrohr (50):
**Entfetten** (105) der inneren Oberfläche (54) des Isolationsrohrs (50).

8. Verfahren nach einem der Ansprüche 4 bis 7, bei dem
das Erwärmen (107) des Schaftbauteils (40) ein Erzeugen eines orts- und zeitabhängigen **Temperaturprofils** entlang des Schaftbauteils (40) mit einem Maximum bei oder über einer Schmelztemperatur oder einer Übergangstemperatur oder einer Erweichungstemperatur des elektrisch isolierenden Materials des Isolationsrohrs (50) umfasst,
die Position des Maximums des Temperaturprofils entlang des Schaftbauteils (40) bewegt wird.

9. Verfahren nach einem der Ansprüche 4 bis 8, bei dem
das Heizen des Schaftbauteils (40) ein Erzeugen (107) eines Wirbelstroms in dem Schaftbauteil (40) umfasst.

10. Verfahren nach einem der Ansprüche 4 bis 9, bei dem
das Schaftbauteil ein **Schaftrohr** (40) mit einem Lumen (42) ist,
das Erwärmen (107) des Schaftbauteils ein Zuführen von Leistung zu einer in dem Lumen (42) des Schaftrohrs (40) angeordneten Heizeinrichtung (70) und ein Bewegen der Heizeinrichtung (70) relativ zu dem Schaftrohr (40) entlang des Schaftrohrs (40) umfasst.
